# EUROPEAN PATENT SPECIFICATION

(11) **EP 4 551 093 B1**
(45) Date of publication and mention of the grant of the patent: **26.11.2025**
(21) Application number: 23738027.4
(22) Date of filing: 04.07.2023
(51) Int. Cl.: A61B 5/00, A61B 5/11

(54) **A SYSTEM AND METHOD FOR DETERMINING A SLEEP POSTURE OF A USER DURING A SLEEP SESSION**
SYSTEM UND VERFAHREN ZUM BESTIMMEN EINER SCHLAFPOSITION EINES BENUTZERS WÄHREND EINER SCHLAFSITZUNG
SYSTÈME ET PROCÉDÉ PERMETTANT DE DÉTERMINER UNE POSTURE DE SOMMEIL D'UN UTILISATEUR PENDANT UNE SESSION DE SOMMEIL

(30) Priority: 05.07.2022 US 202263358504 P; 14.07.2022 EP 22184843
(43) Date of publication of application: 14.05.2025
(73) Proprietor: Signify Holding B.V., 5656 AE Eindhoven (NL)
(72) Inventor: YU, Jin, 5656 AE Eindhoven (NL); DEIXLER, Peter, 5656 AE Eindhoven (NL)
(74) Representative: Verweij, Petronella Daniëlle
(86) International application number: PCT/EP2023/068324
(87) International publication number: WO 2024/008687

(56) References cited:
- WO-A1-2020/145130
- JP-A- 2020 113 243
- CHEN ZHANGJIE ET AL: "Remote Recognition of In-Bed Postures Using a Thermopile Array Sensor With Machine Learning", IEEE SENSORS JOURNAL, IEEE, USA, vol. 21, no. 9, 16 February 2021 (2021-02-16), pages 10428 - 10436, XP011848590, ISSN: 1530-437X, [retrieved on 20210402], DOI: 10.1109/JSEN.2021.3059681

## Description

### FIELD OF THE INVENTION

The invention relates to a system for determining a sleep posture of a user during a sleep session. The invention further relates to a method, a controller, a computer program product for determining a sleep posture of a user during a sleep session.

### BACKGROUND

Monitoring of sleep postures is important for many health-related applications. For example, for tracking the progression of Parkinson's disease, for detecting dementia, for alerting users experiencing potentially fatal (e.g., sleeping on the stomach may increase the risk of sudden infant death syndrome) and/or undesirable sleep postures, for determining sleep quality, for preventing bedsores in bedridden users, etc.

For example, Z. Chen and Y. Wang, "Remote Recognition of In-Bed Postures Using a Thermopile Array Sensor With Machine Learning," discloses determining in-bed postures of a user using a thermopile array sensor.

### SUMMARY OF THE INVENTION

State-of-art solutions for monitoring sleep postures include camera-based systems that may pose privacy and legal considerations, accelerometer-based wearable sensors that may be obtrusive for the user, pressure sensors embedded in the user's bedsheets which may have high deployment cost, etc. Simpler sensors like Single Pixel Thermopile (SPT) sensors, acoustic sensor, PIR sensors, etc., can be used for monitoring sleep postures. Even though the application of such simple sensors may be highly attractive due to costs, the limited capabilities, limited resolution, and accuracy of such sensors have made it challenging for sleep posture monitoring applications. Therefore, it is an object to provide an improved method for determining the sleep posture of a user based on simple, low-resolution, and low-cost sensors, such as Single Pixel Thermopiles, PIRs, etc.

According to a first aspect, the object is achieved by a system for determining a sleep posture of a user during a sleep session, the system comprising: one or more sensors from a plurality of sensors configured to measure a signal indicative of a motion of the user; and a controller configured to receive the signals from the one or more sensors indicative of a motion of the user; determine a turnover event from a predetermined plurality of reference turnover events based on the received signal; determine the sleep posture of the user from a predetermined plurality of reference sleep postures based on the turnover events.

The inventors have realized that sleep postures do not independently evolve over time but are conditioned on transition (tumover) events between the sleep postures. For example, users typically adopt/settle into a sleep posture for a period of time, where the body orientation is approximately constant, then a transition motion (tumover event) follows, after which the user settles into a different sleep posture. That is, a turnover (transition) event may be associated with the turning body transition motion between reference sleep postures. Thus, detecting such transition (tumover) events between the different sleep postures may be used to determine (infer) the sleep posture that the user has adopted / settled into. Since there may be a limited number of predetermined reference sleep, there can be a limited number of predetermined reference turnover events associating transitions between the reference sleep postures. For example, the reference sleep postures may comprise facing up, facing down, facing right, facing left, etc. and the reference turn-over events may comprise a turnover event from facing up to right, turnover event from facing up to left, etc. Each reference turnover event may be associated with a transition motion between two reference sleep postures. The reference sleep postures may be set (predetermined) in advance, for example, the reference sleep postures may be predetermined based on the sensing capabilities of the sensors, a user input, i.e., or the user may be able to select the reference sleep postures via a user interface, a history of sleeping postures of the user, etc.

Low-cost sensors such as Single Pixel Thermopile (SPT) sensors, PIR and microphone sensors, etc., may be used to determine the (turning body) motion associated with such transition (tumover) events and based thereupon to determine the sleep status of the user. For example, time-series signals from acoustic sensors may be used to determine the characteristic pattern of the body motion associated with turning from one reference sleep posture to another. This allows for an improved method for determining the sleep posture of a user based on simple, low-cost sensors.

The one or more sensors comprise Single Pixel Thermopile (SPT), wherein each single pixel thermopile sensor from the one or more sensors is configured to measure a temperature signal in its respective Field-of-View indicative of a motion of the user. Time-series signals from SPT sensors may be used to detect the (turning body) motion associated with such transition (tumover) events associating transitions between the different reference sleep postures, e.g., transition motion from reference sleep posture of lying facing up to reference sleep posture of lying on the side facing to the right. Thus, the one or more sensors from the plurality of sensors may comprise single pixel thermopile sensors, wherein each single pixel thermopile sensor may be configured to measure a temperature signal indicative of a motion of the user in its respective Field-of-View. The measured temperature of an SPT sensor depends on the angle of incidence of the user within the detection area of the SPT. Thus, the measured temperature of an SPT sensor may change when an object, e.g., the user, moves towards or away from the SPT sensor. For example, a user facing towards the SPT may render a higher temperature value in the temperature signal compared to a user facing at an angle from the SPT. Thus, when the user transitions from one sleep posture to another, the measured temperature signal of the SPT sensor follows a similar transition. For example, when the user moves from facing towards (0° angle) the SPT to facing at 90° angle to the SPT, the measured temperature time-series signal will (after an initial rise due to the movement) settle to a lower average temperature value compared to the average temperature value when the user was facing towards the sensor. Thus, the time-series signals from SPT sensors may be used to determine the distinguishable motion (turnover) events associated with transitions between reference sleep postures. The sleep posture may then be determined based on the turnover events. The time-series signal may refer to a single instance in time (e.g., temperature values at a single instance) or a short window \ segment, or period in time, e.g., a 0.5 second, a 1-minute period in time, etc.

The controller may be configured to receive a first and a second signal from a first and a second sensor respectively from the plurality of sensors and the determination of the turnover event is based at least partially on a comparison of the first signal to the second signal. When a single sensor is used to determine such turnover events, the accuracy of the system may be limited. For example, a single SPT sensor located such that the center of its Field-of-View faces towards the user (user's bed) may be subject to thermal interference and thus may not distinguish between a turnover event of 90° or -90° (turning clockwise or anticlockwise). The controller may be configured to receive a first and a second signal from a first and a second sensor respectively, at a same moment in time, each comprising a respective Field-of-View, and determine the turnover event at least partially based on a comparison of the first signal to the second signal. A moment in time may refer to a real-time moment in time or a time instance / window, or period of time in the past. Comparing the first and the second (differential) signals from the two different sensors, each with center of Field-of-View facing the user from different angles allows to determine the unique signal pattern associated with each turnover event and thus mitigates any possible thermal interference.

The controller may be configured to determine (classify) a turnover event from a plurality of predetermined reference turnover events by applying a machine learning model, such as a Support Vector Machine, Logistic regression model, a neural network model, or a probabilistic model on the received signals. The machine learning model may be trained using a training dataset comprising as input instances (time-series data) of the signals received over a time segment and as output, labeled instances of reference turnover events. This enables to directly determine a turnover event while using less computational resources.

The machine learning model may be configured to output a probability value for each of the plurality of reference turnover events, said probability value being the probability of the received signals belonging to each of the predetermined plurality of reference turnover events, and wherein the controller may be configured to compare the probability values of each predetermined reference turnover events, and determine the turnover event based on the comparison. **In** certain situations, determining the probability (value) that the received signal belongs to each of the plurality of reference turnover events and determining the turnover event based on the estimated probabilities may be desirable. This offers more information compared to directly determining (classifying) the turnover event without producing the probability estimation. The machine learning model may be a probabilistic model and may have been trained to output a probability value for each of the plurality of reference turnover events, said probability value being the probability of the received signals belonging to each of the predetermined plurality of reference turnover events. The controller may be configured to determine a turnover event from a plurality of reference turnover events by comparing the probabilities of the received signals belonging to each of the predetermined plurality of reference turnover events.

The controller may be configured to determine a current likelihood value for each of the predetermined plurality of reference sleep postures at a current moment in time based on
- the determined probability values of each of the predetermined plurality of turnover events at the current moment in time and
- the past likelihood values for each of the predetermined plurality of reference sleep postures determined in a previous moment in time; and
   wherein the controller is further configured to
- compare the current likelihood values of each predetermined reference sleep postures, and
- determine the sleep posture at the current moment in time based on the comparison.

A sleep session may be seen as a chronological sequence of sleep postures and transitions (tumovers events) between the sleep postures. For example, a user may initially be facing up, then make a 90° turn and transition from facing up to facing to the right side, settle into the right position for a period of time and make another 90° turn and transition from facing to the right to facing down, etc. Since a sleep posture may have only two possible transition motions associated with it, i.e., clockwise (90° turn) and anticlockwise (-90° turn), the sleep posture that the user may have settled into at a moment in time may depend on the previous (preceding) sleep posture at a previous (preceding) moment in time and the transition motion associating the previous sleep posture with the current sleep posture at the current moment in time. A moment in time may be a single instance in time or a short window \ segment, or period in time, e.g., a 0.5 second, a 1-minute period in time, etc. The controller may be configured to determine a second likelihood value for each of the predetermined plurality of reference sleep postures at a second moment in time based on the determined probability values of each of the predetermined plurality of reference turnover events at the second moment in time and the past likelihood values of each of the predetermined plurality of reference sleep postures determined in a preceding moment in time. A preceding moment in time may refer to the moment in time associated with the previously received signal. The controller may determine the sleep posture at the second moment in time by comparing the second likelihood values of each predetermined reference sleep posture. This allows for a probabilistic approach to determine the sleep posture at a moment in time based on the determined turnover event at that moment in time and the history of previous (preceding) sleep posture estimations.

The controller may be configured to select a subset of the plurality of sensors based on the field of view of the plurality of sensors for monitoring the sleep posture of the user. **In** a typical home or elderly care setting, a plurality of sensors may be available at different relative locations, each having a different field of view relative to the user (user's bed). It may be advantageous to select a subset of the plurality of available sensors at different locations for monitoring the sleep posture of the user based on the field of view of the sensors. For example, a combination of an SPT sensor in the ceiling and an SPT sensor at the side of the user, e.g., bed-side desk, may offer (less overlapping) field of view and have a good coverage (no information loss) of the sleeping person. This subset selection allows for optimal separability between the sleep posture classes. Thus, selecting a subset of the plurality of sensors at different relative locations allows for more accurate sleep posture estimation.

The controller may be configured to determine a position from a set of predefined positions of the one or more sensors based on the Field-of-view of the one or more sensors relative to a position of the user, and wherein the controller is further configured to output the determined position of the one or more sensors to the user. **In** certain cases, a limited number of sensors configured to measure a signal indicative of a motion of the user may be available. It would be advantageous to inform the user on the optimal (preferred) position to install (position) the one or more (limited) sensors. For example, each sensor may be part of a luminaire. The user may be informed on which position from a set of predefined positions to install the luminaires (and thus the sensor). In an embodiment, a set of predefined positions of the one or more sensors may be available, e.g., the user may define the set of predefined possible positions (e.g., luminaire positions) in a floor map through a user interface. The controller may be configured to determine a position of the one or more sensors from the set of predefined reference positions based on the Field-of-view of the one or more sensors relative to a position of the user. The controller may further be configured to output, for example to a target device, the preferred determined positions of the one or more sensors. This allows to inform the user on the optimal positions of the sensors, such that the user can optimally configure the sleep posture system.

Certain health conditions and diseases may be associated with spending time in specific reference sleep postures. For example, sleeping facing down (lying on the stomach) may lead to chronic pain and nerve issues and may be associated with a high risk of sudden death syndrome in toddlers, sleeping facing up may be correlated with sleep apnea and snoring, sleeping on the right side has a higher risk than sleeping on the left side of development of transient lower esophageal sphincter relaxation, which is a main factor in nocturnal gastroesophageal reflux, etc. The controller may be configured to determine if the determined sleep posture of the user is an undesirable sleep posture. That is, a sleep posture that may be associated with certain health conditions, e.g., apnea, and/or may result in pain, such as neck, back pain, headaches, if the user remains for a prolonged predetermined period of time in the undesirable sleep posture. The controller may further be configured to determine the period of time elapsed after the detection of the undesired sleep posture. This offers a way to prevent or minimize the time that the user may spend time in an undesirable sleep posture. The processor may further be configured to output an alert signal after a predetermined period of time has elapsed after the detection of the undesirable sleep posture. The alert signal may be a signal that induces a change in the user's sleep posture, for instance the alert signal may inform the user to change the sleep posture from facing down lying on the stomach to facing to the side (right or left). The alert signal may be in the form of an optical signal, e.g., a luminaire may be turned on. Alternatively, or additionally, the alert signal may be a sound signal, e.g., an alarm from an alarm clock, a vibration signal, etc. By outputting such an alert signal to the user, the user may be informed that (s)he may have spent a lengthy (predetermined) period of time in an undesirable sleep posture and induce a change in his or her sleep posture.

Sleep quality may be related to sleep posture and frequent sleep postural changes. For example, frequent sleep posture changes may be associated with a lower sleep quality index, snoring and frequent body movements may result in a shorter sleep duration, sleeping in face-down position may strain the neck and lower back, etc. Furthermore, sleeping on the left may be correlated to an increased rate of nightmares, sleeping facing-up may be correlated to sleep apnea, headaches, etc. Patients with Parkinson's disease often suffer from loss of axial movement; and less frequent nocturnal turnovers and longer periods spent recumbent or supine (i.e., facing up postures) are associated with deterioration in the condition of Parkinson's patients. The controller may further be configured to determine a frequency of turnover events during a sleep session, i.e., the number of determined turnover events during a sleep session. The controller may further be configured to determine a sleep quality index based on at least one of the sleep related parameters: the period of time elapsed in undesirable sleep postures for the user and/or the frequency of turnover events. For example, a sleep quality index may be determined by assigning a score value to the sleep-related parameters of the user. For example, each parameter (period of time in undesirable sleep posture(s) for the user, frequency (number) of turnover events during sleep, duration of sleep, etc.) may be given a score based on their value compared to an optimal value for the user. Initially, the optimal values against which the user parameters are scored may be determined by normal population results.

The controller may be configured to receive sleep-related data from one or more further sensors from the plurality of sensors, determine based on the received sleep-related data if the sleep status of the user is an asleep sleep status, and determine the sleep posture of the user based on the condition that the sleep status of the user is an asleep status. The inventors recognize that the accuracy of methods for determining the progress of various mental and/or heath diseases, e.g., Parkinson's disease, dementia, etc., based on sleep posture data may be hampered if the sleep posture determination method includes instances where the user is not asleep. Hence, it is advantageous to condition (initiate) the sleep posture detection system upon the detection that the user is asleep. The controller may be configured to receive sleep-related data from one or more further sensors from the plurality of sensors and to determine based on the received sleep-related data if the sleep status of the user is an asleep sleep status. The controller may determine the sleep posture of the user only based on the condition of the determination of an asleep status of the user.

The controller may further be configured to receive self-annotating instances of sleep postures and/or turnover events by the user and train a machine learning model at least in part on the self-annotating sleep postures. A user interface may provide an indication of or allow the user to specify (self-annotate) his \ her sleep postures during one or more past sleep sessions. For example, the user interface may provide to the user a presentation of video recordings of one or more past sleep sessions and may provide the user means to self-annotate instances of the video recordings, i.e., self-annotate instances of reference turnover events, self-annotate instances of sleep postures, etc. The processor may be configured to receive the self-annotating instances of sleep postures and/or turnover events by the user and train a machine-learning model at least in part based on the self-annotating sleep postures. For example, train a machine-learning model using inputs instances (time-series data) of temperature signals over time segments of the one or more past sleep sessions and as output the self-annotating instances of reference turnover events. Additionally, or alternatively, the processor may determine the turnover events based on the self-annotating instances of sleep postures.

According to a second aspect, the object is achieved by a method for determining a sleep posture of a user during a sleep session, the method comprising the steps of: receiving signals from a plurality of sensors indicative of a motion of the user, determining a turnover event from a predetermined plurality of reference turnover events based on the received signals, and determining the sleep posture of the user from a predetermined plurality of reference sleep postures based on the turnover events.

According to a third aspect, the object is achieved by a controller for determining a sleep posture of a user during a sleep session.

According to a fourth aspect, the object is achieved by a computer program product for a computing device, the computer program product comprising computer program code to perform the method for determining a sleep posture of a user during a sleep session.

It should be understood that the controller, method and computer program product may have similar and/or identical embodiments and advantages as the above-mentioned lighting devices.

### BRIEF DESCRIPTION OF THE DRAWINGS

The above, as well as additional objects, features and advantages of the disclosed systems, devices and methods will be better understood through the following illustrative and non-limiting detailed description of embodiments of devices and methods, with reference to the appended drawings, in which:
Fig. 1 shows schematically an example of a system for determining a sleep posture of a user;
Fig. 2 shows schematically an example of a system for determining a sleep posture of a user;
Fig. 3 shows schematically a flow diagram of reference sleep postures and reference turnover events associated with transitions between reference sleep postures;
Fig. 4 shows schematically an example of a system for determining a sleep posture of a user;
Fig. 5 shows schematically a flow diagram of a method of determining a sleep posture of a user.

All the figures are schematic, not necessarily to scale, and generally only show parts which are necessary in order to elucidate the invention, wherein other parts may be omitted or merely suggested.

### DETAILED DESCRIPTION

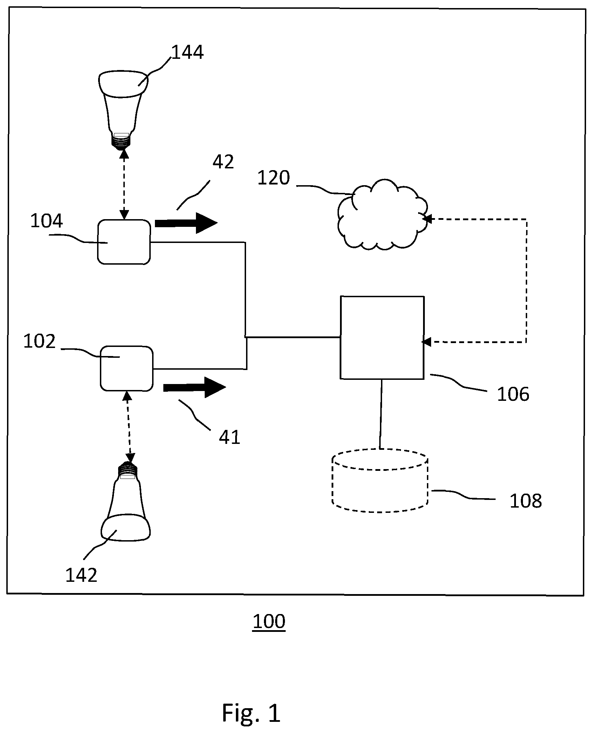

Figure 1 shows an example of system 100 for determining a sleep posture of a user (not shown). The system 100 comprises one or more sensors from a plurality of sensors 102, 104 configured to measure a signal indicative of a motion of a user. The one or more sensors 102, 104 may for example be Single Pixel Thermopile sensors configured to measure a temperature signal in their respective Field-of-View. Additionally, or alternatively, the one or more sensors 102, 104 may be (single array) infrared (IR) sensors configured to capture reflections of the projected IR light pattern from an object in an environment, acoustic sensor configured to capture acoustic signals from an environment, etc. In an example, the one or more sensors 102, 104 may be co-located with lighting devices 142, 144.

The system 100 further comprises at least one data processor or controller 106. The controller 106 may be in connection and communication with each sensor 102, 104 via a wireless connection, via e.g., a radiofrequency or an optical communication link. For example, Wi-Fi, ZigBee, BLE, Lo-Ra, UWB, VLC, IR, Li-Fi, etc., connection. Said connection may alternatively be wired.

Referring to Figure 1, each sensor 102, 104 may comprise a transmitter (not depicted) for transmitting (or: providing) the respective signal 41, 42 to the controller 106 via the wired or wireless connection. The controller 106 may comprise a receiver (not depicted) for receiving each respective signal 41, 42 from the respective sensor 102, 104. The system 100 may further comprise at least one data repository or storage or memory 108 for storing computer program code instructions.

Yet alternatively, the system 100 may comprise a server. Each sensor 102, 104 may convey their respective signal 41, 42 to the server, such that the server may obtain each respective signal 41, 42. The controller 106 may then be configured to retrieve (receive) each respective signal 41, 42 from the server. The controller 106 may be communicatively coupled to the cloud 120.

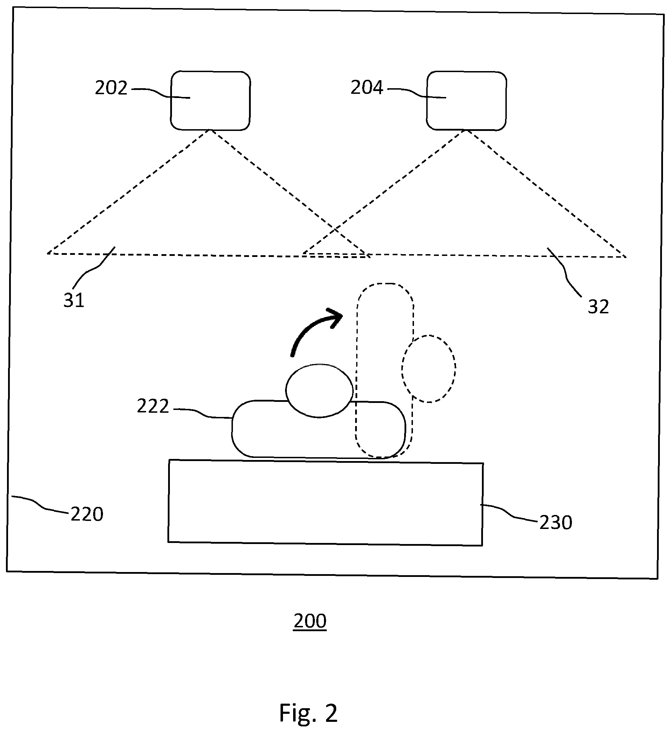

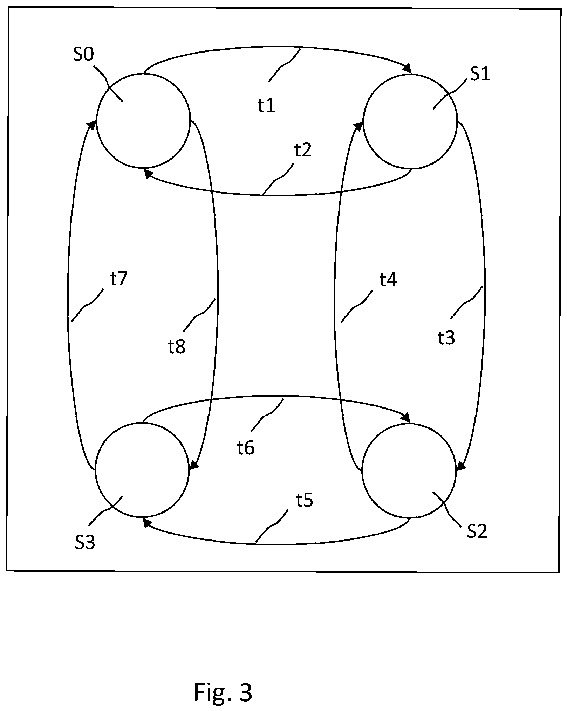

Referring to Figure 2, the system 200 according to the invention may be arranged within a space 220. The space may for example be a bedroom, a hospital room, etc. The bedroom, hospital room, etc., may comprise a bed 230 where the user 222 typically sleeps. With reference to Figure 3, a user 222 may adopt a limited number of reference sleep postures, for example reference sleep postures may include face up S0, face down S2, right-lateral S1, left-lateral S3. A turning motion of the user 222 (turnover event) may be associated with the transitions between the different sleep postures. Each reference turnover event t1-t8 may be associated with a transition (change in the orientation of the user 222) between one reference sleep posture to another. For example, the reference turnover event t1 is associated with the transition from facing up S0 to facing to the right S1. The turnover event t2 is associated with the reverse transition, namely transition from facing to the right S1 to facing up S0. The reference turnover event t3 is associated with the transition from facing to the right S1 to facing down S2, etc.

Now referring back to Figure 2, the system 200 is arranged for determining the sleep posture of the user 222 during a sleeping session within the space 220. The first sensor 202 may be a SPT sensor that comprises a first Field-of-View 31 that (partially) covers the user. The second sensor 204 may be a SPT sensor that comprises a second Field-of-View 32 that (partially) covers that user, the second Field-of-View 32 may be different from the first Field of View 31 at least by a threshold). Here, each respective Field-of-View 31, 32 is similar and circular around its projection (or: central) axis. At least part of each respective Field-of-View 31, 32 of each sensor 202, 204 covers the user's 222 bed 230. As mentioned before, a SPT sensor may only measure a single temperature value within its respective (whole) Field-of-View over time. Hence, each SPT sensor 202, 204 of the two SPT sensors measures a temperature signal 41, 42 in its respective Field-of-View 31, 32. The temperature signal may, in examples, be phrased differently as a heat signal.

An insight of the present invention is that the value of a measured temperature signal of an SPT sensor, even though it may measure a single temperature for its whole Field-of-View, may be indicative of the distance of a detected person to the center of the Field-of-View of the SPT sensor. For example, a person settling at a sleep posture facing closer to the center of the Field-of-View of a particular SPT sensor may (at the same moment in time) render a higher temperature value in the temperature signal of this particular SPT sensor compared to said person settling at a sleep posture facing away from the center of the Field-of-View of this particular SPT sensor. Moreover, said moment in time may not be a single instance in time, but a relatively short window, segment, or period in time. Hence, said moment in time may be a period in time having a duration of at most 0.5 second, or at most 0.1 second.

Thus, the time-series temperature signals 41, 42 may change when the user 222 moves towards or away from the sensors 202, 204. For example, when the user 222 sleeps facing up S0, the temperature signals 41, 42 measured by the first sensor and the second sensor 102 respectively, 104 show an average value I0. When the user 222 makes a rotating motion of 90°, i.e., turnover event t1 from reference sleep posture face up S0 to facing towards the right, lateral-right sleep posture S1, the temperature signal 41 from the first sensor 202 initially rises due to the turning motion and then settles into an average temperature I1 that is lower than I0 since the user 222 adopts a sleep posture S0 that faces away from the center of the Field-of-View of sensor 202. In comparison, the temperature signal 42 from the first sensor 204 initially rises due to the turning motion and then settles into an average temperature I2 that is higher than I0 since the user 222 adopts a sleep posture S0 that faces towards the center of the Field-of-View of sensor 204. Thus, the controller may determine the turnover event t1 from the predetermined reference turnover events t1-t8 based on the received signals 41, 42.

In another example, when the user 222 sleeps facing down S2, the temperature signals 41, 42 measured by the first sensor and the second sensor 202 respectively, 204 show an average value I0. When the user 222 makes a rotating motion of 90°, i.e., turnover event t5 from reference sleep posture face down S2 to lateral-left sleep posture S3, the temperature signals 41, 42 from both the first and the second sensor will initially increase (with different step size, overshoot, etc.) due to the turning motion of the user 222. After the initial increase, when the user has settled into the lateral-left sleep posture S3, both the temperature signals 41, 42 from both the first and the second sensor will settle into higher temperature values I1, I2 than I0, however, the temperature value I1 of the first sensor 202 will have a higher value compared to the temperature value I2 of the second sensor 204, since the user 222 adopts a sleep posture S3 that faces towards the center of the Field-of-View of sensor 202. Thus, the controller may determine the turnover event t5 from the predetermined reference turnover events t1-t8 based on the received signals 41, 42.

The size of steps in temperature, the overshoot and average temperature preceding and succeeding a turnover event, and other statistical features of the signals 41, 42 such as rise time, etc., are correlated with the turnover associating the transition from one reference sleep posture to another. Thus, controller 106 may determine (classify) a turnover event from the plurality of predetermined reference turnover events at a current moment in time based on the received signals 41, 42 from sensors 202, 204 based on the correlation of the signals 41, 42 with the turnover events. For example, the controller 106 may compare the temperature signal 41 to the temperature signal 42 and based on this comparison determine the turnover event.

Additionally, or alternatively, the controller 106 may be configured to determine the turnover event of the user 222 by directly applying a machine-learning model, such as SVM, neural network model, logistic regression model, etc., on the received signals 41, 42. For example, the received signals 41, 42 from the sensors 202, 204 may be used as input to the machine learning model to determine the turnover event from the plurality of reference turnover events t1-t8 of the user 222. The trained machine learning model may make such a determination because the machine learning model may have already been trained with inputs that may include instances or segments (time series data) of signals 41, 42 received from sensors 202, 204 within the space 220 and output corresponding labeled instances of the refence turnover events t1-t8.

Additionally, or alternatively, the controller 106 may be configured to determine the probability of a turnover event, i.e., a probability the received signals belong to each of the predetermined plurality of reference turnover events t1-t8 by applying a probabilistic machine learning model, such as a Gaussian mixture model, a Bayesian neural network model, etc., on the received signals 41, 42. The trained probabilistic machine learning model may make such a determination because the machine learning model may have already been trained to input signals 41, 42 and output a probability of the received signals 41, 42 belonging to each of the reference turnover events t1-t8.

The controller 106 may be configured to determine the sleep posture of the user 222 from a predetermined plurality of reference sleep postures S0-S3 based on the turnover events. For example, with reference to Figure 2, the controller 106 may determine the current sleep posture S1 based on the determination of the turnover event t1 that is associated with the transition motion from sleep posture S0 in the previous moment in time to sleep posture S1 in the current moment in time.

Additionally, or alternatively, the controller 106 may be configured to determine the likelihood of each of the predetermined reference sleep postures S0-S3 at a moment in time and determine the sleep posture by comparing the likelihoods of each of the reference sleep postures, e.g., by applying a maximum a posteriori probability (MAP) rule. For example, the controller 106 may determine the current (second) likelihood of each of the predetermined reference sleep postures S0-S3 based on the determined probabilities of the received signals 41, 42 belonging to each of the predetermined plurality of turnover events at that moment in time and the likelihoods for each of the predetermined plurality of reference sleep postures determined in a previous (preceding) moment in time. For example, the second likelihood determination (at a current moment in time) may be based on a posterior probability calculation, i.e., the likelihood of a reference sleep posture at a (second) moment in time is proportional to the addition of the likelihoods of being in a sleep posture at a preceding moment in time (preceding likelihoods), times the probability that you transition from the preceding sleep posture to the new one, i.e., the probability of the turnover events.

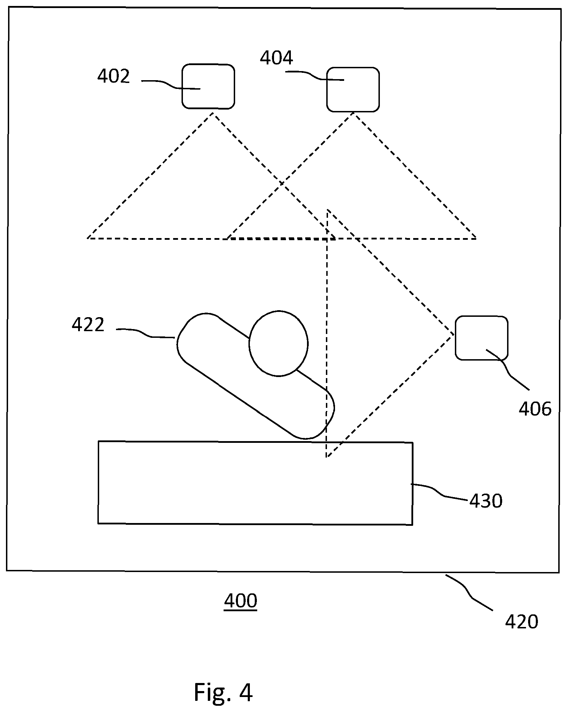

With reference to Figure 4, a space 420 may comprise a plurality of sensors 402, 404, 406, at different relative locations, each having a different field of view relative to the user's bed 430. The controller 106 may be configured to select a subset of the plurality of sensors 402, 404, 406 based on their respective field of view towards the user. For example, the controller 106 may select a subset 402, 406 of the plurality of sensors 402, 404, 406 as they offer (less overlapping) field of view and have a good coverage (no information loss) of the sleeping user 422.

The controller 106 may be configured to determine if the sleep posture of the user 222 is an undesirable sleep posture and to determine a period of time elapsed after the detection of the undesirable sleep posture. The controller 106 may be configured to output an alert signal after a predetermined period of time has elapsed after the detection of the undesirable sleep posture. The alert signal may be in the form of an optical signal, e.g., a luminaire may be turned on. Alternatively, or additionally, the alert signal may be a sound signal, e.g., an alarm from an alarm clock, a vibration signal, etc. In another example, the alert signal may be a message signal, such as a text message to a mobile device, a notification in a user interface, etc. Alternatively, the alert signal as previously described may be output to a third party, e.g., a parent, caregiver, etc., if the sleep posture of the user 222 is an undesirable sleep posture.

The controller 106 may further be configured to receive sleep-related data from one or more further sensors from the plurality of sensors and determine if the sleep status of the user is asleep based on the received sleep-related data. For example, the one or more further sensors may be acoustic sensors configured to generate digitized breath sounds recorded from the user 222 over time and the controller 106 may determine the sleep status of the user 222 (sleep status may be on of: awake or asleep) based on the digitized breath sounds recorded from the user 222 over time. In another example, the one or more further sensors may be Radio Frequency (RF) nodes configured transmit and received RF signals and the controller 106 may be configured to determine the respiration rate of the user 222 based on the received RF signals. The controller 106 may further be configured to determine if the sleep status of the user is asleep based on the respiration rate of the user 222. Methods and techniques for determining the start of a sleeping session are known in the art and will therefore not be discussed in further detail. The controller 106 may further be configured to determine the sleep posture of the user 222 only upon the determination of an asleep status of the user 222. That is, the method steps of the sleep posture determination are implemented only based on the condition that the sleep status of the user is an asleep status.

The controller 106 may be configured to receive self-annotating instances of sleep postures and/or turnover events by the user 222, e.g., through a user interface, and train a machine-learning model at least in part based on the self-annotating sleep postures. For example, train a machine-learning model using inputs instances (time-series data) of temperature signals 41, 42 over time segments of the one or more past sleep sessions of the user 222 and as output the self-annotating instances of reference turnover events. Additionally, or alternatively, the processor 106 may annotate the turnover events by associating the self-annotating instances of sleep postures.

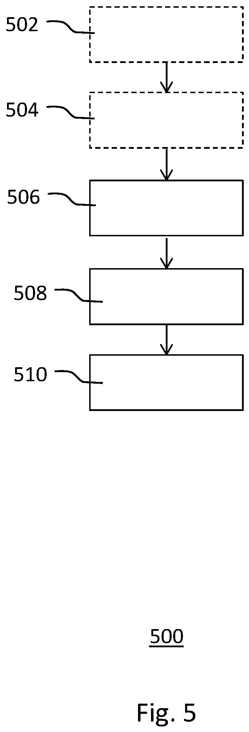

Figure 5 shows a method 500 of determining a sleep posture of a user, the method comprising the steps of:
- receiving 502 sleep-related data;
- determining 504 if the sleep status of the user is asleep based on the received sleep-related data;
- receiving 506 signals 41, 42 from one or more sensors 202, 204 from a plurality of sensors indicative of a motion of the user;
- determining 508 a turnover event from a predetermined plurality of reference turnover events t1-t8 based on the received signals;
- determining 510 the sleep posture of the user 222 from a predetermined plurality of reference sleep postures S0-S3 based on the turnover events. The steps 502, 504 may be optional.

The method 500 may be executed by computer program code of a computer program product when the computer program product is run on a processing unit of a computing device, such as the processor/controller 106.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim. Use of the verb "comprise", and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer or processing unit. In the device claim enumerating several means, several of these means may be embodied by one and the same item of hardware. The mere fact that certain measures are recited in mutually different dependent claims does not indicate that a combination of these measures cannot be used to advantage.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g., updates) or extensions for existing programs (e.g., plugins). Moreover, parts of the processing of the present invention may be distributed over multiple computers or processors or even the 'cloud'.

Storage media suitable for storing computer program instructions include all forms of nonvolatile memory, including but not limited to EPROM, EEPROM and flash memory devices, magnetic disks such as the internal and external hard disk drives, removable disks and CD-ROM disks. The computer program product may be distributed on such a storage medium, or may be offered for download through HTTP, FTP, email or through a server connected to a network such as the Internet.

## Claims

1. A system (100) for determining a sleep posture of a user during a sleep session, wherein the system comprises:
- two or more sensors (102, 104) from a plurality of sensors configured to measure a signal indicative of a motion of the user, said sensors comprising single pixel thermopile sensors and/or infrared sensors; and
- a controller (106) configured to
receive the signals from the two or more sensors indicative of a motion of the user;
determine a turnover event from a predetermined plurality of reference turnover events based on the received signals, said predetermined plurality of reference turnover events comprising one or more of facing up to facing right (t1), facing up to facing left (t8), facing down to facing right (t4), facing down to facing left (t5), facing left to facing up (t7), facing left to facing down (t6), facing right to facing down (t3), and facing right to facing up (t2);
determine the sleep posture of the user from a predetermined plurality of reference sleep postures based on the turnover events, said predetermined plurality of reference sleep postures comprising one or more of facing up (S0), facing down (S2), facing right lateral (S1), and facing left lateral (S3).

2. The system according to claim 1, wherein the controller is configured to receive a first and a second signal from a first and a second sensor respectively from the plurality of sensors and wherein the determination of the turnover event is based at least partially on a comparison of the first signal to the second signal.

3. The system according to claim 1 or 2, wherein the controller is configured to determine the turnover event by applying a machine learning model on the received signals, wherein the machine learning model has been trained on the received signals indicative of a motion of the user.

4. The system according to claim 3, wherein the machine learning model is a probabilistic model configured to output a probability value for each of the plurality of reference turnover events, said probability value being the probability of the received signals belonging to each of the predetermined plurality of reference turnover events, and wherein the controller may be configured to:
- compare the probability values of each predetermined reference turnover events; and
- determine the turnover event based on the comparison.

5. The system according to claim 4, wherein the controller is configured to determine a second likelihood value for each of the predetermined plurality of reference sleep postures at a second moment in time based on:
- the determined probability values of each of the predetermined plurality of turnover events at the second moment in time and
- the past likelihood values for each of the predetermined plurality of reference sleep postures determined in a first moment in time, wherein the first moment in time is preceding the second moment in time; and
wherein the controller is further configured to:
- compare the second likelihood values of each predetermined reference sleep posture, and
- determine the sleep posture at the second moment in time based on the comparison.

6. The system according to claim 1, wherein the controller is configured to select a subset of the plurality of sensors based on the field of view of the plurality of sensors for monitoring the sleep posture of the user.

7. The system according to any of the preceding claims, wherein the controller is configured to determine a position from a set of predefined positions of the one or more sensors based on the Field-of-view of the one or more sensors relative to a position of the user, and wherein the controller is further configured to output the determined position of the one or more sensors to the user.

8. The system according to claim 1, wherein the controller is further configured to determine:
- if the determined sleep posture of the user is an undesirable sleep posture;
- determine a period of time elapsed after the detection of the undesirable sleep posture; and
- output an alert signal after a predetermined period of time has elapsed after the detection of the undesirable sleep posture.

9. The system according to claim 8, wherein the controller is further configured to:
- determine a frequency of turnover events during a sleep session;
- determine a sleep quality index based on the period of time elapsed in an undesirable sleep posture and/or the frequency of turnover events.

10. The system according to any of the preceding claims, wherein the controller is configured to:
- receive sleep-related data from one or more further sensors from the plurality of sensors and
- determine based on the received sleep-related data if the sleep status of the user is an asleep sleep status; and
- determine the sleep posture of the user based on the condition that the sleep status of the user is an asleep status.

11. The system according to claim 3, wherein the controller is further configured to: receive self-annotating instances of sleep postures and/or turnover events by the user, and wherein the machine learning model is trained at least in part on the self-annotating sleep postures.

12. A controller (106) for determining a sleep posture of a user during a sleep session, the controller configured to:
- receive signals from the two or more sensors (102, 104) indicative of a motion of the user, said sensors comprising single pixel thermopile sensors and/or infrared sensors;
- determine a turnover event from a predetermined plurality of reference turnover events based on the received signals, said predetermined plurality of reference turnover events comprising one or more of facing up to facing right (t1), facing up to facing left (t8), facing down to facing right (t4), facing down to facing left (t5), facing left to facing up (t7), facing left to facing down (t6), facing right to facing down (t3), and facing right to facing up (t2);
- determine the sleep posture of the user from a predetermined plurality of reference sleep postures based on the turnover events, said predetermined plurality of reference sleep postures comprising one or more of facing up (S0), facing down (S2), facing right lateral (S1), and facing left lateral (S3).

13. A method (500) for determining a sleep posture of a user during a sleep session, the method comprising the steps of:
- receiving (506) signals from two or more sensors from a plurality of sensors indicative of a motion of the user, said sensors comprising single pixel thermopile sensors and/or infrared sensors;
- determining (508) a turnover event from a predetermined plurality of reference turnover events based on the received signals, said predetermined plurality of reference turnover events comprising one or more of facing up to facing right (t1), facing up to facing left (t8), facing down to facing right (t4), facing down to facing left (t5), facing left to facing up (t7), facing left to facing down (t6), facing right to facing down (t3), and facing right to facing up (t2);
- determining (510) the sleep posture of the user from a predetermined plurality of reference sleep postures based on the turnover events, said predetermined plurality of reference sleep postures comprising one or more of facing up (S0), facing down (S2), facing right lateral (S1), and facing left lateral (S3).

14. A computer program product comprising instructions which, when the program is executed by a computer, cause the computer to carry out the steps of the method of claim 13.

## Patentansprüche

1. System (100) zum Bestimmen einer Schlafhaltung eines Benutzers während einer Schlafsitzung, wobei das System umfasst:
- zwei oder mehr Sensoren (102, 104) von einer Vielzahl von Sensoren, die konfiguriert sind, um ein Signal zu messen, das eine Bewegung des Benutzers anzeigt, die Sensoren umfassend Einzelpixel-Thermosäulensensoren und/oder Infrarotsensoren umfassen; und
- eine Steuerung (106), die konfiguriert ist zum
Empfangen der Signale von den zwei oder mehr Sensoren, die eine Bewegung des Benutzers anzeigen;
Bestimmen eines Umdrehereignisses von einer zuvor bestimmten Vielzahl von Referenzumdrehereignissen basierend auf den empfangenen Signalen, die zuvor bestimmte Vielzahl von Referenzumdrehereignissen umfassend eines oder mehrere von nach oben gerichtet zu nach rechts gerichtet (t1), nach oben gerichtet zu nach links gerichtet (t8), nach unten gerichtet zu nach rechts gerichtet (t4), nach unten gerichtet zu nach links gerichtet (t5), nach links gerichtet zu nach oben gerichtet (t7), nach links gerichtet zu nach unten gerichtet (t6), nach rechts gerichtet zu nach unten gerichtet (t3) und nach rechts gerichtet zu nach oben gerichtet (t2);
Bestimmen der Schlafhaltung des Benutzers von einer zuvor bestimmten Vielzahl von Referenzschlafhaltungen basierend auf den Umdrehereignissen, die zuvor bestimmte Vielzahl von Referenzschlafhaltungen umfassend eines oder mehrere von nach oben gerichtet (S0), nach unten gerichtet (S2), nach rechts seitlich gerichtet (S1) und nach links seitlich gerichtet (S3).

2. System nach Anspruch 1, wobei die Steuerung konfiguriert ist, um ein erstes und ein zweites Signal von einem ersten beziehungsweise einem zweiten Sensor von der Vielzahl von Sensoren zu empfangen, und wobei die Bestimmung des Umdrehereignisses mindestens teilweise auf einem Vergleich des ersten Signals mit dem zweiten Signal basiert.

3. System nach Anspruch 1 oder 2, wobei die Steuerung konfiguriert ist, um das Umdrehereignis durch ein Anwenden eines maschinellen Lernmodells auf die empfangenen Signale zu bestimmen, wobei das maschinelle Lernmodell anhand der empfangenen Signale trainiert wurde, die eine Bewegung des Benutzers anzeigen.

4. System nach Anspruch 3, wobei das maschinelle Lernmodell ein Wahrscheinlichkeitsmodell ist, das konfiguriert ist, um für jedes der Vielzahl von Referenzumdrehereignissen einen Wahrscheinlichkeitswert auszugeben, wobei der Wahrscheinlichkeitswert die Wahrscheinlichkeit ist, dass die empfangenen Signale zu jedem der zuvor bestimmten Vielzahl von Referenzumdrehereignissen gehören, und wobei die Steuerung konfiguriert sein kann zum:
- Vergleichen der Wahrscheinlichkeitswerte aller zuvor bestimmten Referenzumdrehereignisse; und
- Bestimmen des Umdrehereignisses basierend auf dem Vergleich.

5. System nach Anspruch 4, wobei die Steuerung konfiguriert ist, um einen zweiten Wahrscheinlichkeitswert für jede der zuvor bestimmten Vielzahl von Referenzschlafhaltungen zu einem zweiten Zeitpunkt zu bestimmen, basierend auf:
- den bestimmten Wahrscheinlichkeitswerten jedes der zuvor bestimmten Vielzahl von Umdrehereignissen zu dem zweiten Zeitpunkt und
- den vergangenen Wahrscheinlichkeitswerten für jede der zuvor bestimmten Vielzahl von Referenzschlafhaltungen, die zu einem ersten Zeitpunkt bestimmt werden, wobei der erste Zeitpunkt dem zweiten Zeitpunkt vorausgeht; und
wobei die Steuerung ferner konfiguriert ist zum:
- Vergleichen der zweiten Wahrscheinlichkeitswerte jeder zuvor bestimmten Referenzschlafhaltung und
- Bestimmen der Schlafhaltung zu dem zweiten Zeitpunkt basierend auf dem Vergleich.

6. System nach Anspruch 1, wobei die Steuerung konfiguriert ist, um eine Teilmenge der Vielzahl von Sensoren basierend auf dem Sichtfeld der Vielzahl von Sensoren zum Überwachen der Schlafhaltung des Benutzers auszuwählen.

7. System nach einem der vorstehenden Ansprüche, wobei die Steuerung konfiguriert ist, um eine Position von einem Satz von vordefinierten Positionen des einen oder der mehreren Sensoren basierend auf dem Sichtfeld des einen oder der mehreren Sensoren relativ zu einer Position des Benutzers zu bestimmen, und wobei die Steuerung ferner konfiguriert ist, um die bestimmte Position des einen oder der mehreren Sensoren an den Benutzer auszugeben.

8. System nach Anspruch 1, wobei die Steuerung ferner konfiguriert ist zum Bestimmen:
- ob die bestimmte Schlafhaltung des Benutzers eine unerwünschte Schlafhaltung ist;
- Bestimmen einer Zeitspanne, die nach der Erkennung der unerwünschten Schlafhaltung vergangen ist; und
- Ausgeben eines Warnsignals nach Ablauf einer zuvor bestimmten Zeitspanne nach der Erkennung der unerwünschten Schlafhaltung.

9. System nach Anspruch 8, wobei die Steuerung ferner konfiguriert ist zum:
- Bestimmen einer Häufigkeit von Umdrehereignissen während einer Schlafsitzung;
- Bestimmen eines Schlafqualitätsindex basierend auf der Zeitspanne, die in einer unerwünschten Schlafhaltung vergangen ist, und/oder der Häufigkeit von Umdrehereignissen.

10. System nach einem der vorstehenden Ansprüche, wobei die Steuerung konfiguriert ist zum:
- Empfangen von schlafbezogenen Daten von einem oder mehreren weiteren Sensoren von der Vielzahl von Sensoren und
- Bestimmen basierend auf den empfangenen schlafbezogenen Daten, ob der Schlafzustand des Benutzers ein schlafender Schlafzustand ist; und
- Bestimmen der Schlafhaltung des Benutzers basierend auf der Bedingung, dass der Schlafzustand des Benutzers ein schlafender Zustand ist.

11. System nach Anspruch 3, wobei die Steuerung ferner konfiguriert ist zum: Empfangen von selbstannotierenden Instanzen von Schlafhaltungen und/oder Umdrehereignissen durch den Benutzer, und wobei das maschinelle Lernmodell mindestens teilweise anhand der selbstannotierenden Schlafhaltungen trainiert wird.

12. Steuerung (106) zum Bestimmen einer Schlafhaltung eines Benutzers während einer Schlafsitzung, wobei die Steuerung konfiguriert ist zum:
- Empfangen von Signalen von den zwei oder mehr Sensoren (102, 104), die eine Bewegung des Benutzers anzeigen, die Sensoren umfassend Einzelpixel-Thermosäulensensoren und/oder Infrarotsensoren;
- Bestimmen eines Umdrehereignisses von einer zuvor bestimmten Vielzahl von Referenzumdrehereignissen basierend auf den empfangenen Signalen, die zuvor bestimmte Vielzahl von Referenzumdrehereignissen umfassend eines oder mehrere von nach oben gerichtet zu nach rechts gerichtet (t1), nach oben gerichtet zu nach links gerichtet (t8), nach unten gerichtet zu nach rechts gerichtet (t4), nach unten gerichtet zu nach links gerichtet (t5), nach links gerichtet zu nach oben gerichtet (t7), nach links gerichtet zu nach unten gerichtet (t6), nach rechts gerichtet zu nach unten gerichtet (t3) und nach rechts gerichtet zu nach oben gerichtet (t2);
- Bestimmen der Schlafhaltung des Benutzers von einer zuvor bestimmten Vielzahl von Referenzschlafhaltungen basierend auf den Umdrehereignissen, die zuvor bestimmte Vielzahl von Referenzschlafhaltungen umfassend eines oder mehrere von nach oben gerichtet (S0), nach unten gerichtet (S2), nach rechts seitlich gerichtet (S1) und nach links seitlich gerichtet (S3).

13. Verfahren (500) zum Bestimmen einer Schlafhaltung eines Benutzers während einer Schlafsitzung, das Verfahren umfassend die Schritte:
- Empfangen (506) von Signalen von zwei oder mehr Sensoren von einer Vielzahl von Sensoren, die eine Bewegung des Benutzers anzeigen, die Sensoren umfassend Einzelpixel-Thermosäulensensoren und/oder Infrarotsensoren;
- Bestimmen (508) eines Umdrehereignisses von einer zuvor bestimmten Vielzahl von Referenzumdrehereignissen basierend auf den empfangenen Signalen, die zuvor bestimmte Vielzahl von Referenzumdrehereignissen umfassend eines oder mehrere von nach oben gerichtet zu nach rechts gerichtet (t1), nach oben gerichtet zu nach links gerichtet (t8), nach unten gerichtet zu nach rechts gerichtet (t4), nach unten gerichtet zu nach links gerichtet (t5), nach links gerichtet zu nach oben gerichtet (t7), nach links gerichtet zu nach unten gerichtet (t6), nach rechts gerichtet zu nach unten gerichtet (t3) und nach rechts gerichtet zu nach oben gerichtet (t2);
- Bestimmen (510) der Schlafhaltung des Benutzers von einer zuvor bestimmten Vielzahl von Referenzschlafhaltungen basierend auf den Umdrehereignissen, die zuvor bestimmte Vielzahl von Referenzschlafhaltungen umfassend eines oder mehrere von nach oben gerichtet (S0), nach unten gerichtet (S2), nach rechts seitlich gerichtet (S1) und nach links seitlich gerichtet (S3).

14. Computerprogrammprodukt, umfassend Anweisungen, die, wenn das Programm durch einen Computer ausgeführt wird, den Computer veranlassen, die Schritte des Verfahrens nach Anspruch 13 vorzunehmen.

## Revendications

1. Système (100) permettant de déterminer une posture de sommeil d'un utilisateur pendant une session de sommeil, dans lequel le système comprend :
- deux capteurs (102, 104) ou plus parmi une pluralité de capteurs configurés pour mesurer un signal indiquant un mouvement de l'utilisateur, lesdits capteurs comprenant des capteurs à thermopile à pixel unique et/ou des capteurs infrarouges ; et
- un organe de commande (106) configuré pour
recevoir les signaux provenant des deux capteurs ou plus indiquant un mouvement de l'utilisateur ;
déterminer un événement de retournement parmi une pluralité prédéterminée d'événements de retournement de référence en fonction des signaux reçus, ladite pluralité prédéterminée d'événements de retournement de référence comprenant un ou plusieurs parmi de tourné vers le haut à tourné vers la droite (t1), de tourné vers le haut à tourné vers la gauche (t8), de tourné vers le bas à tourné vers la droite (t4), de tourné vers le bas à tourné vers la gauche (t5), de tourné vers la gauche à tourné vers le haut (t7), de tourné vers la gauche à tourné vers le bas (t6), de tourné vers la droite à tourné vers le bas (t3), et de tourné vers la droite à tourné vers le haut (t2) ;
déterminer la posture de sommeil de l'utilisateur à partir d'une pluralité prédéterminée de postures de sommeil de référence en fonction des événements de retournement, ladite pluralité prédéterminée de postures de sommeil de référence comprenant un ou plusieurs parmi tourné vers le haut (S0), tourné vers le bas (S2), tourné vers le côté droit (S1), et tourné vers le côté gauche (S3).

2. Système selon la revendication 1, dans lequel l'organe de commande est configuré pour recevoir un premier et un second signal en provenance d'un premier et d'un second capteur respectivement parmi la pluralité de capteurs et dans lequel la détermination de l'événement de retournement est en fonction au moins partiellement d'une comparaison du premier signal au second signal.

3. Système selon la revendication 1 ou 2, dans lequel l'organe de commande est configuré pour déterminer l'événement de retournement en appliquant un modèle d'apprentissage automatique sur les signaux reçus, dans lequel le modèle d'apprentissage automatique a été entraîné sur les signaux reçus indiquant un mouvement de l'utilisateur.

4. Système selon la revendication 3, dans lequel le modèle d'apprentissage automatique est un modèle probabiliste configuré pour délivrer en sortie une valeur de probabilité pour chacun parmi la pluralité d'événements de retournement de référence, ladite valeur de probabilité étant la probabilité que les signaux reçus appartiennent à chacun parmi la pluralité prédéterminée d'événements de retournement de référence, et dans lequel l'organe de commande peut être configuré pour :
- comparer les valeurs de probabilité de chaque événement de retournement de référence prédéterminé ; et
- déterminer l'événement de retournement en fonction de la comparaison.

5. Système selon la revendication 4, dans lequel l'organe de commande est configuré pour déterminer une seconde valeur de vraisemblance pour chacune parmi la pluralité prédéterminée de postures de sommeil de référence à un second moment dans le temps en fonction :
- des valeurs de probabilité déterminées de chacun parmi la pluralité prédéterminée d'événements de retournement au second moment dans le temps et
- des valeurs de vraisemblance passées pour chacune parmi la pluralité prédéterminée de postures de sommeil de référence déterminées à un premier moment dans le temps, dans lequel le premier moment dans le temps précède le second moment dans le temps ; et
dans lequel l'organe de commande est configuré en outre pour :
- comparer les secondes valeurs de vraisemblance de chaque posture de sommeil de référence prédéterminée, et
- déterminer la posture de sommeil au second moment dans le temps en fonction de la comparaison.

6. Système selon la revendication 1, dans lequel l'organe de commande est configuré pour sélectionner un sous-ensemble de la pluralité de capteurs en fonction du champ de vision de la pluralité de capteurs permettant de surveiller la posture de sommeil de l'utilisateur.

7. Système selon l'une quelconque des revendications précédentes, dans lequel l'organe de commande est configuré pour déterminer une position parmi un ensemble de positions prédéfinies du ou des capteurs en fonction du champ de vision du ou des capteurs par rapport à une position de l'utilisateur, et dans lequel l'organe de commande est configuré en outre pour délivrer en sortie la position déterminée du ou des capteurs à l'utilisateur.

8. Système selon la revendication 1, dans lequel l'organe de commande est configuré en outre pour déterminer :
- si la posture de sommeil déterminée de l'utilisateur est une posture de sommeil indésirable ;
- déterminer un laps de temps écoulé après la détection de la posture de sommeil indésirable ; et
- délivrer en sortie un signal d'alerte après qu'un laps de temps prédéterminé s'est écoulé après la détection de la posture de sommeil indésirable.

9. Système selon la revendication 8, dans lequel l'organe de commande est configuré en outre pour :
- déterminer une fréquence d'événements de retournement pendant une session de sommeil ;
- déterminer un indice de qualité de sommeil en fonction du laps de temps écoulé dans une posture de sommeil indésirable et/ou de la fréquence d'événements de retournement.

10. Système de commande selon l'une quelconque des revendications précédentes, dans lequel l'organe de commande est configuré pour :
- recevoir des données apparentées au sommeil en provenance d'un ou plusieurs capteurs supplémentaires parmi la pluralité de capteurs et
- déterminer en fonction des données apparentées au sommeil reçues si l'état de sommeil de l'utilisateur est un état de sommeil endormi ; et
- déterminer la posture de sommeil de l'utilisateur en fonction de la condition que l'état de sommeil de l'utilisateur est un état endormi.

11. Système selon la revendication 3, dans lequel l'organe de commande est configuré en outre pour : recevoir des instances auto-annotées de postures de sommeil et/ou d'événements de retournement par l'utilisateur, et dans lequel le modèle d'apprentissage automatique est entraîné au moins en partie sur les postures de sommeil auto-annotées.

12. Organe de commande (106) permettant de déterminer une posture de sommeil d'un utilisateur pendant une session de sommeil, l'organe de commande étant configuré pour :
- recevoir des signaux en provenance des deux capteurs (102, 104) ou plus indiquant un mouvement de l'utilisateur, lesdits capteurs comprenant des capteurs à thermopile à pixel unique et/ou des capteurs infrarouges ;
- déterminer un événement de retournement parmi une pluralité prédéterminée d'événements de retournement de référence en fonction des signaux reçus, ladite pluralité prédéterminée d'événements de retournement de référence comprenant un ou plusieurs parmi de tourné vers le haut à tourné vers la droite (t1), de tourné vers le haut à tourné vers la gauche (t8), de tourné vers le bas à tourné vers la droite (t4), de tourné vers le bas à tourné vers la gauche (t5), de tourné vers la gauche à tourné vers le haut (t7), de tourné vers la gauche à tourné vers le bas (t6), de tourné vers la droite à tourné vers le bas (t3), et de tourné vers la droite à tourné vers le haut (t2) ;
- déterminer la posture de sommeil de l'utilisateur à partir d'une pluralité prédéterminée de postures de sommeil de référence en fonction des événements de retournement, ladite pluralité prédéterminée de postures de sommeil de référence comprenant un ou plusieurs parmi tourné vers le haut (S0), tourné vers le bas (S2), tourné vers le côté droit (S1), et tourné vers le côté gauche (S3).

13. Procédé (500) permettant de déterminer une posture de sommeil d'un utilisateur pendant une session de sommeil, le procédé comprenant les étapes consistant à :
- recevoir (506) des signaux en provenance de deux capteurs ou plus parmi une pluralité de capteurs indiquant un mouvement de l'utilisateur, lesdits capteurs comprenant des capteurs à thermopile à pixel unique et/ou des capteurs infrarouges ;
- déterminer (508) un événement de retournement parmi une pluralité prédéterminée d'événements de retournement de référence en fonction des signaux reçus, ladite pluralité prédéterminée d'événements de retournement de référence comprenant un ou plusieurs parmi de tourné vers le haut à tourné vers la droite (t1), de tourné vers le haut à tourné vers la gauche (t8), de tourné vers le bas à tourné vers la droite (t4), de tourné vers le bas à tourné vers la gauche (t5), de tourné vers la gauche à tourné vers le haut (t7), de tourné vers la gauche à tourné vers le bas (t6), de tourné vers la droite à tourné vers le bas (t3), et de tourné vers la droite à tourné vers le haut (t2) ;
- déterminer (510) la posture de sommeil de l'utilisateur à partir d'une pluralité prédéterminée de postures de sommeil de référence en fonction des événements de retournement, ladite pluralité prédéterminée de postures de sommeil de référence comprenant une ou plusieurs parmi tourné vers le haut (S0), tourné vers le bas (S2), tourné vers le côté droit (S1), et tourné vers le côté gauche (S3).

14. Produit programme informatique comprenant des instructions qui, lorsque le programme est exécuté par un ordinateur, amènent l'ordinateur à effectuer les étapes du procédé selon la revendication 13.
